# EUROPEAN PATENT SPECIFICATION

(11) **EP 1 002 566 B1**
(45) Date of publication and mention of the grant of the patent: **21.09.2011**
(21) Application number: 99121976.7
(22) Date of filing: 10.11.1999
(51) Int. Cl.: B01D 63/02, B01D 63/10, A61M 1/16, A61M 1/36, B01D 15/08

(54) **Filter for adsorbing heparin and/or endotoxins in plasma and/or blood**
Filter geeignet zur Adsorption von Heparin und/oder Endtoxinen in Plasma und/oder Blut
Filtre pour l'adsorption d' héparine et/ou d'endotoxine dans du plasma et/ou du sang

(30) Priority: 12.11.1998 IT MO980235
(43) Date of publication of application: 24.05.2000
(73) Proprietor: MAT Adsorption Technologies GmbH & Co. KG, 63785 Obernburg (DE)
(72) Inventor: Bellini, Gianni, 41036 Medolla (Prov. of Modena) (IT)
(74) Representative: Schröder, Richard

(56) References cited:
- EP-A- 0 165 568
- EP-A- 0 341 413
- EP-A- 0 466 178
- WO-A-88/06899
- WO-A-98/28064

## Description

The present invention relates to a filter for adsorbing heparin and/or endotoxins in plasma and/or blood.

In most clinical treatments in which blood or biological fluids must be treated according to specific procedures related to the disease of a patient, the extracorporeal circuit in which these fluids flow must receive the addition of anticoagulants, such as for example heparin, in order to contain the allergic reaction which, as it is known, endogenous fluids develop in contact with foreign surfaces or materials.

Relatively high doses of this substance are therefore normally used in treatments such as blood oxygenation, in cardiopulmonary bypass, hemodialysis, hemofiltration, hemoperfusion and plasmapheresis.

In the end of these extracorporeal treatments, or in case of hemorrhages or complications occurred during the treatment, it is necessary to neutralize as quickly as possible the heparin that is still circulating in the cardiovascular system and might be present in high concentrations; protamine sulfate is used in many of these cases as a heparin antagonist.

However, this method has some limitations due to the patient's failure to metabolize part of the volumes of heparin used and to a consequent resumption of release of the heparin several hours after its apparent neutralization, especially in the case of high administered doses.

Moreover, the use of heparin is not limited to the anticoagulant action that it produces; in fact it can also be employed in an acid environment with a pH between 5.05 and 5.25, for therapeutic purposes for the selective removal of lipoproteins (cholesterol) such as LDL in dislipidemic patients.

In this regard, the prior art describes in detail a process for the extracorporeal precipitation and selective separation of low-density lipoproteins (LDL) known as HELP (Heparin Extracorporeal LDL Precipitation) if heparin is added to the plasma of the patient so as to form complexes of insoluble heparin-LDL aggregates which can be filtered.

According to this process, which uses an acetate buffer solution containing heparin in an amount equivalent to 100 UI/ml, the plasma of the patient is placed in contact with this solution in a 1:1 volume ratio, obtaining the precipitation of LDL, fibrinogen, LP(a) and B-100 apoprotein in clinically effective quantities.

Since heparin is added by means of a buffer solution in an amount which exceeds the actual binding capacity of these proteins, it is important to remove the excess quantity in order to re-establish the initial conditions of the plasma, avoiding the increase in risk of hemorrhage and the possible above-mentioned complications.

This removal is currently performed by using filters which have cellulose or cellulose-based membranes and have a substantially bulky structure with respect to the actual filtration yield.

These filters have also significant problems as regards sterilization before their use: in fact it is necessary to use incompatible liquids for this operation and in order to keep the filtering membrane intact, and the subsequent elimination of these liquids requires washing with physiological solution, which however does not ensure full elimination of traces of these toxic liquids, which might therefore be infused to the patient.

Finally, another problem shared by all conventional filters is the lack of uniform distribution of the blood or plasma along the filtering surfaces: the flows of blood or plasma in fact tend to wet only the proximal portions thereof, whereas the distal ones remain substantially unaffected.

WO-A98/28064 discloses a filter which comprises in a housing having an inlet port connected to a distribution chamber and an outlet port connected to a collection chamber an arrangement of first and second hollow-fiber membranes. According to this document, first and second hollow-fiber membranes are embedded with their ends in sealing compounds or packing elements such that the ends of the first hollow-fiber membranes facing the distribution chamber are embedded in a sealing compound adjourning the distribution chamber at the inlet end of the housing such that they are protruding this sealing compound and are open towards the distribution chamber. The ends of the second hollow-fiber membranes facing the collection chamber are embedded in a sealing compound adjourning the collection chamber at the outlet end of the housing such that they are protruding this sealing compound adjourning the collection chamber and are open towards the collection chamber. The hollow-fiber membranes may be arranged in multiple layers which are wrapped concentrically in a roll and may be modified with ligands such as diethylaminoethyl groups.

The aim of the present invention is to solve the above-cited problems of the prior art by providing a filter for adsorbing heparin and/or endotoxins in blood and/or plasma which is capable of eliminating said substances substantially completely and allows to optimize the distribution of blood and plasma on the entire area of the filtering surfaces and at the same time avoid reduction of the filtering action caused by possible clogging.

This aim, object and others are achieved by a filter for adsorbing heparin and/or endotoxins in blood and/or plasma, comprising an internally hollow cylindrical containment body which is provided, at its inlet and outlet ends, with corresponding access ports formed in end caps and internally accommodating a filtering medium composed of microporous fibers having open ends and being arranged in multiple layers which are wrapped concentrically in a roll and have groups of diethylaminoethyl deposited thereon, characterized in that the filter accomodates a single filtering medium only composed of microporous fibers and in that a packing element for stable packing of the ends of the fibers is provided at the outlet end only of said body, wherein the open ends of all of the microporous fibers are embedded at the outlet end of said body in the packing element so as to pass through it.

Further characteristics and advantages will become apparent from the description of a preferred embodiment of a filter for adsorbing heparin and/or endotoxins from blood and/or plasma, illustrated only by way of non-limitative example in the accompanying drawings, wherein:

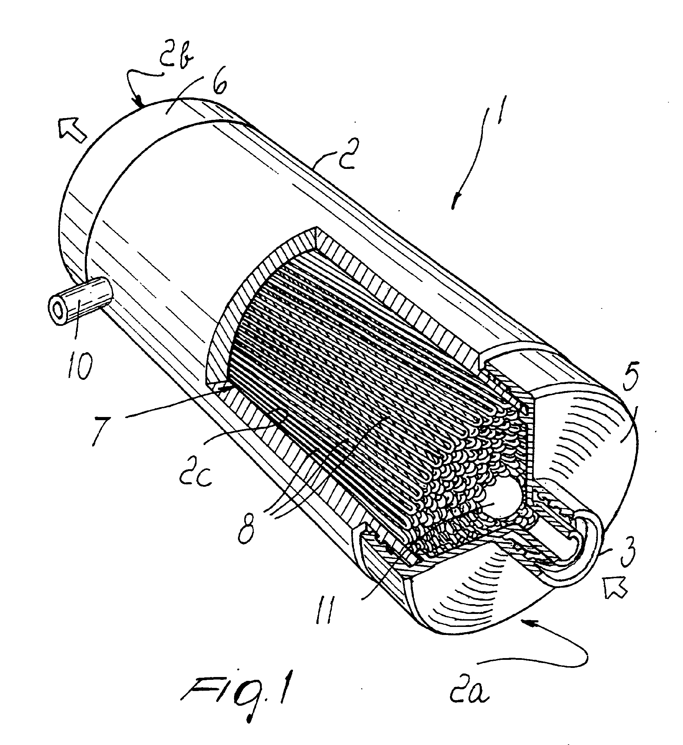

Figure 1 is a partially cutout perspective view of the adsorbent filter according to the invention;

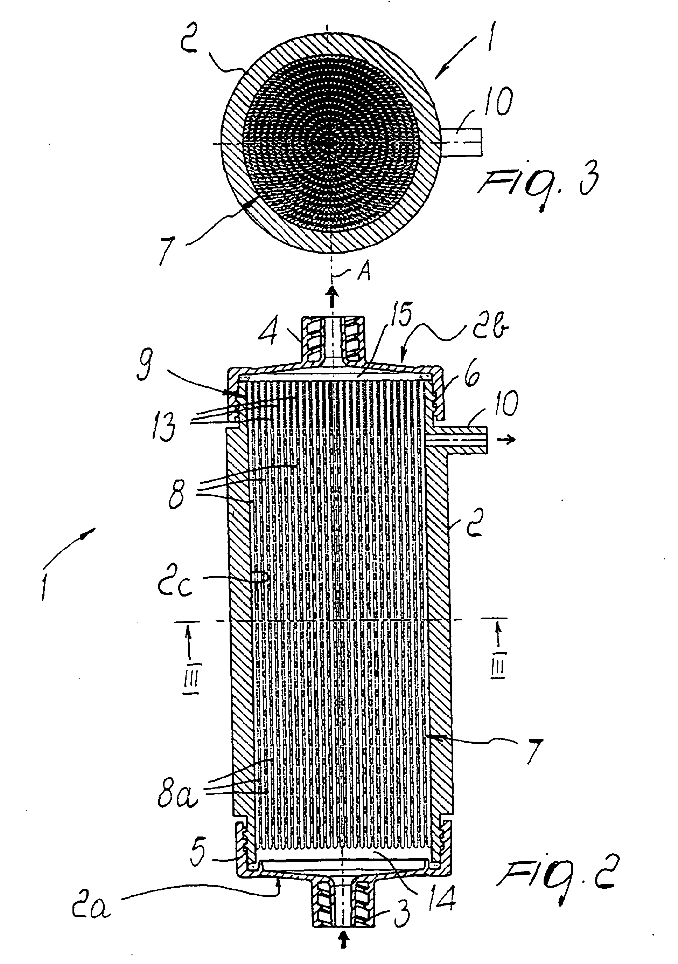

Figure 2 is a longitudinal sectional view thereof;
Figure 3 is a corresponding transverse sectional view thereof, taken along the plane III-III of Figure 2;

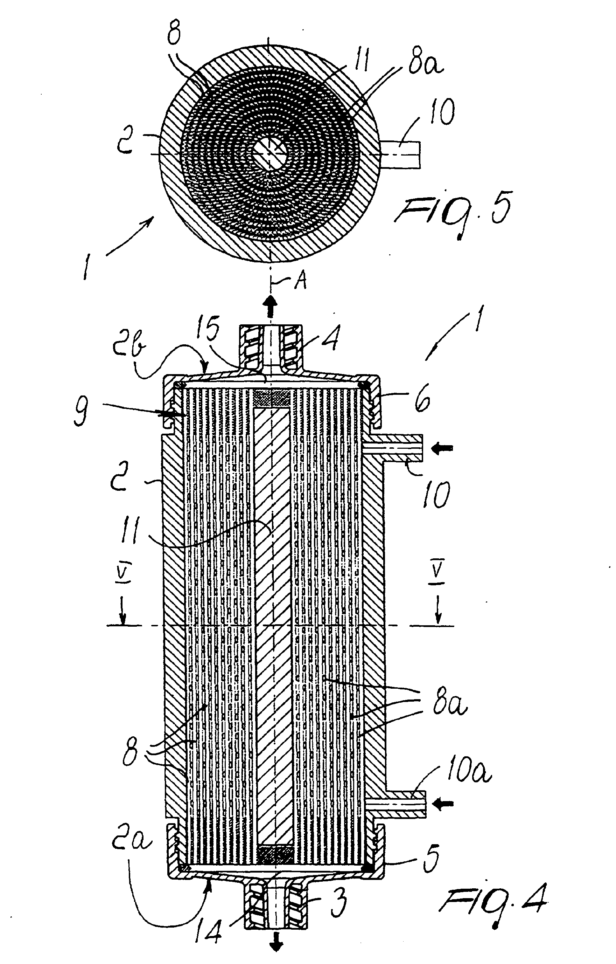

Figure 4 is a longitudinal sectional view of an adsorbent filter not according to the invention;
Figure 5 is a corresponding transverse sectional view thereof, taken along the plane V-V of Figure 4;

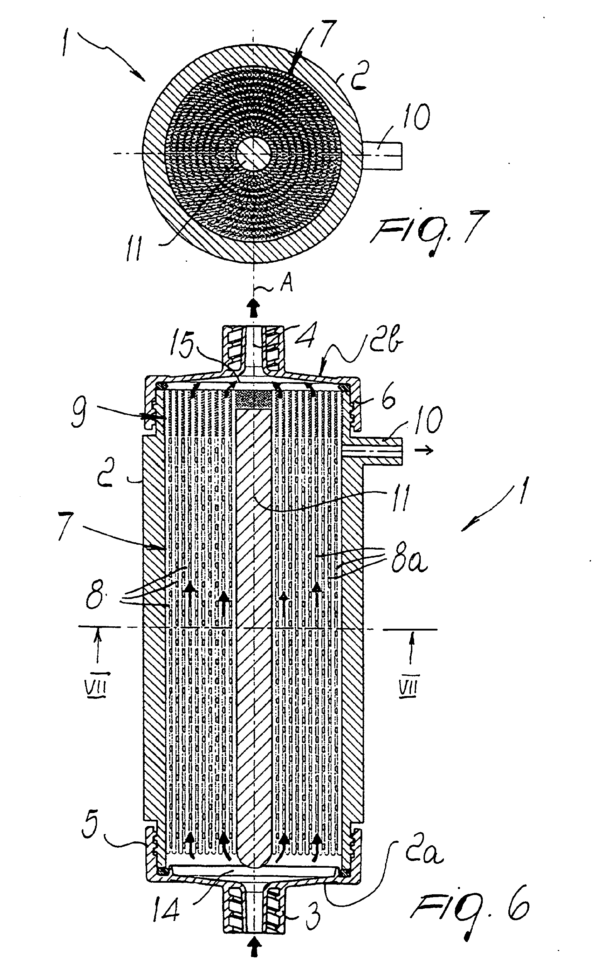

Figure 6 is another longitudinal sectional view of the invention in a second possible embodiment;
Figure 7 is a corresponding transverse sectional view thereof, taken along the plane VII-VII of Figure 6;
Figure 8 is a front view of a filtering medium with straight microporous fibers;
Figure 9 is another front view of a filtering medium with inclined microporous fibers;
Figure 10 is a detail view of a step of the packaging of a medium with stratified microporous fibers.

With reference to the above figures, the reference numeral 1 designates a filter for adsorbing heparin and/or endotoxins in blood and/or plasma which essentially comprises a cylindrical containment body 2 which is internally hollow and is provided, at its inlet end 2a and at its outlet end 2b, with corresponding access ports, designated by the reference numerals 3 and 4 respectively, which are centered and coaxial with respect to the containment body 2 in end caps 5 and 6.

The internal cavity 2c of the containment body 2 accommodates a filtration medium 7 composed of microporous fibers 8 made of polyamide which are stratified in multiple layers and are concentrically wound in a roll and on which diethylaminoethyl groups are deposited; moreover, at least at the outlet end 2b of the body there is, in an internal region, an element 9 for the stable packing of the heads of the fibers 8.

In addition to the ports 3 and 4, in the containment body 2 there is a third port 10 for quick draining arranged laterally thereto proximate to the outlet end 2b.

In a preferred embodiment of the filter 1 there is a longitudinal cylindrical rib 11 centered in the internal axial cavity of the containment body 2; the filtering medium 7 with microporous fibers 8 can be wrapped around the rib and is supported by it.

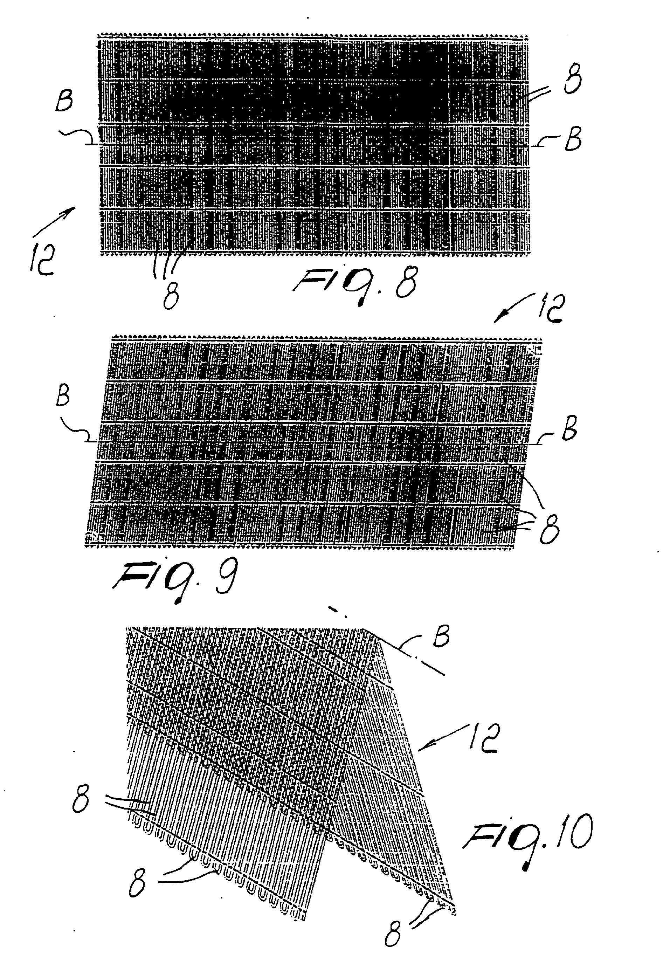

In practice, said medium is constituted by a woven sheet of membrane 12 (see for example Figures 8 and 9) whose weft is composed of said mutually particularly microporous fibers 8; the woven sheet is then packaged in a roll and the fibers 8 accordingly arrange themselves in a zigzag fashion; the roll is then placed snugly in the axial cavity of the containment body 2, optionally fitted on the rib 11.

The woven sheet 12 is usually rectangular and can be coupled to at least one second substantially identical rectangular woven sheet which however is made of a material whose weft has nonporous and parallel fibers 8a made of polyethylene or polypropylene, polyester or plastic known by the tradename Nylon: the packaging of said woven sheets in a roll causes an alternating concentric stratification of the microporous fibers 8 and the nonporous fibers 8a.

According to one version of the invention, the parallel microporous fibers 8 that constitute the weft of the woven sheet 12 are arranged at an angle with respect to the longitudinal axis A of the containment body 2, so that packaging in a roll generates a filtration medium 7 with microporous fibers 8 which are stratified and cross each other in a grid-like pattern.

The woven sheet 12 with weft made of microporous fibers 8 can furthermore be folded over along the longitudinal axis B at right angle to the fibers, and if the nonporous-fiber woven sheet 8a is coupled to it, the nonporous fibers are also arranged at an angle with respect to the axis A but are opposite the microporous fibers 8 either in a mirror-symmetrical fashion or in an asymmetrical fashion.

Moreover, depending on the filtration capillarity to be achieved, the diameters of the microporous fibers 8 and of the nonporous fibers 8a can be mutually identical or different, so that during packaging in roll form the smallest of the two can penetrate the interspaces formed between the other fibers.

Finally, the stable packing element 9 is constituted by a static flow control element 13 made of elastomer in which the open ends or heads of the microporous fibers 8 are embedded so as to pass through it; this element is snugly inserted at the outlet end 2b, inside the axial cavity of the containment body 2.

Collection chamber 15 is formed between the static flow control element 13 and the corresponding cap ^ 6, and the blood or plasma that is to be filtered or, respectively, has already been filtered accumulates therein.

The operation of the invention is as follows: the blood or plasma to be filtered, from which in other words the endotoxins or excess heparin must be removed, arriving from the patient and propelled by a slight pressure provided by a conventional type of pumping means arranged along the line, enters the filter 1 through the inlet port 3, fully fills the collection chamber 14 and distributes along the entire internal cavity of the cylindrical containment body 2, wetting the microporous fibers 8 along their entire surface extension uniformly and without any concentration in particular regions of the filter 1.

During this step, possible air that is present between the microporous fibers 8 can be gradually expelled through the lateral port 10; during normal operation of the filter 1, the port 10 is in any case closed.

The diethylaminoethyl groups deposited on the microporous fibers combine chemically with the endotoxin or heparin molecules, producing stable bonds on the surfaces of the microporous fibers 8.

The flow of blood or plasma then passes centripetally and at right angles through the outer surfaces of the microporous fibers 8, and through their capillary openings, reaches the collection chamber 15 and from there, through the port 4, it is returned, purified, to the patient.

The open heads of the microporous fibers 8 are retained in place, while maintaining the open connection to the chamber 15, by the static flow control element 13.

The fibers 8, in order to constitute a highly effective filtration medium 7, are first packaged in the form of a rectangular woven sheet 12, which is then rolled up so that the fibers arrange themselves in a layered arrangement adapted to constitute a filtering grid in a centripetal direction of flow.

The woven sheet 12, before being rolled up, can be subjected to traction at two diagonally opposite corners, so as to incline the fibers 8 at a predefined angle and can be folded about its own median longitudinal axis B in order to produce the grid.

When the woven sheet 12 is rolled up, it can be arranged inside the cavity of the cylindrical body 2 without any support or can be fitted on a longitudinal cylindrical rib 11 provided specifically for this purpose and centered in the cavity.

The woven sheet 12 can further be coupled by parallel faces with a further identical woven sheet whose weft is composed of nonporous fibers 8a: during packaging in roll form, these latter fibers act as inert spacers for the concentric layers of microporous fibers 8.

The diameters of the microporous fibers and of the nonporous fibers 8a can be identical or mutually different and the inclinations can be symmetrical or asymmetric with respect to the longitudinal axis of the filter 1.

In practice it has been observed that the above-described invention achieves the intended aim.

The invention thus conceived is susceptible of several modifications and variations, all of which are within the scope of the inventive concept.

All the details may further be replaced with other technically equivalent ones.

In practice, the materials employed, as well as the shapes and the dimensions, may be any according to requirements without thereby abandoning the scope of the protection of the appended claims.

The disclosures in Italian Patent Application No. MO98A000235 from which this application claims priority are incorporated herein by reference.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A filter for adsorbing heparin and/or endotoxins in blood and/or plasma, comprising an internally hollow cylindrical containment body (2) which is provided, at its inlet (2a) and outlet (2b) ends, with corresponding access ports (3,4) formed in end caps (5,6) and internally accommodating a filtering medium (7) composed of microporous fibers (8) having open ends and being arranged in multiple layers which are wrapped concentrically in a roll and have groups of diethylaminoethyl deposited thereon, **characterized in that** the filter accomodates a single filtering medium (7) only composed of microporous fibers (8) and **in that** a packing element (9) for stable packing of the ends of the fibers (8) is provided at the outlet end (2b) only of said body (2), wherein the open ends of all of the microporous fibers (8) are embedded at the outlet end (2b) of said body (2) in the packing element (9) so as to pass through it.

2. The adsorbent filter according to claim 1, **characterized in that** said access ports (3,4), respectively for the inflow (3) and outflow (4) of blood and/or plasma, are arranged in said caps (5,6) so as to be centered and coaxial with respect to said body (2), a quick draining port (10) being arranged to said body (2) proximate to the outlet end (2b).

3. The adsorbent filter according to claim 1, **characterized in that** a cylindrical longitudinal rib (11) is centered coaxially inside the cavity (2c) formed by said (2) body and is meant for the wrapping and support of said microporous fiber filtering medium (7).

4. The absorbent filter according to claims 1 and 3, **characterized in that** said microporous fiber filtering medium (7) is constituted by a woven sheet of membrane (12) with a weft composed of said microporous membranes (12), which are parallel and folded in a zigzag fashion, packaged in roll form for snug placement in said axial cavity (2c) of said containment body (2) and fitted on said rib (11).

5. The absorbent filter according to claim 4, **characterized in that** said woven sheet of membrane (12) with parallel microporous fiber weft is rectangular and is coupled to at least one second rectangular woven sheet (8a) which is substantially identical and made of material with a parallel nonporous fiber weft, the packaging of said woven sheet (12) in roll form causing an alternating concentric layering of microporous fibers (8) and nonporous fibers (8a).

6. The adsorbent filter according to claim 4, **characterized in that** said woven sheet of membrane (12) with parallel microporous fiber weft is rectangular and is coupled to at least one second identical woven sheet (8a), the packaging of said woven sheets (12) in roll form causing an alternating concentric layering of microporous fibers (8).

7. The adsorbent filter according to the preceding claims, **characterized in that** at least said parallel microporous fibers (8) that constitute the weft of said woven sheet of membrane (12) are arranged at an angle with respect to the longitudinal axis of said containment body (2), said packaging in roll form generating said filtering medium (7) with stratified microporous fibers (8) crossed in a grid-like fashion.

8. The adsorbent filter according to the preceding claims, **characterized in that** said microporous fibers (8) are made of a plastic material chosen among polyamide, polysulfone, polyethersulfone, polyethylene, polypropylene and polyester.

9. The adsorbent filter according to claim 6, **characterized in that** said woven sheet (12) with microporous fiber (8) weft is folded over along the longitudinal axis (B) perpendicular to the fibers (8).

10. The adsorbent filter according to the preceding claims, **characterized in that** said parallel nonporous fibers (8a) are arranged at an angle with respect to the axis (A) of said containment body (2) so as to be opposite, in a mirror-symmetrical fashion, with respect to said microporous fibers (8).

11. The adsorbent filter according to the preceding claims, **characterized in that** said nonporous fibers (8a) are made of a polymeric material chosen among polyethylene, polypropylene, polyester or nylon.

12. The adsorbent filter according to the preceding claims, **characterized in that** said microporous fibers (8) and said nonporous fibers (8a) are arranged at an angle in an asymmetrical opposite fashion.

13. The adsorbent filter according to the preceding claims, **characterized in that** the diameters of said microporous fibers (8) and of said nonporous fibers (8a) are mutually identical.

14. The adsorbent filter according to the preceding claims, **characterized in that** the diameters of said microporous fibers (8) and of said nonporous fibers (8a) are mutually different.

15. The adsorbent filter according to claims 6 and 7, **characterized in that** the inclination of said microporous fibers (8) and of said nonporous fibers (8a) is performed after traction applied to at least two diagonal ends of said woven sheets(12) before being packaged in roll form.

16. The adsorbent filter according to the preceding claims, **characterized in that** said filter medium (7) packaged in roll form has a perimetric surface constituted by microporous fiber (8).

17. The adsorbent filter according to claim 1, **characterized in that** said stable packaging element is constituted by a static flow control element (13) made of an elastomer in which said open ends of the microporous fibers (8) are embedded so as to pass through it, said flow control element (13) being snugly inserted in the outlet end (2b) of the axial cavity (2c) of said cylindrical containment body (2).

18. The adsorbent filter according to claims 1 and 14, **characterized in that** a second static flow control element (13) made of elastomer is inserted also in the inlet end (2a) of the axial cavity (2c) of said cylindrical containment body (2).

19. The adsorbent filter according to claims 1, 14 and 15, **characterized in that** corresponding collection chambers (14, 15) are formed between said caps (5,6) and said static flow control element (13) and return bends of said microporous fibers (8).

## Patentansprüche

1. Filter zum Adsorbieren von Heparin und/oder Endotoxinen in Blut und/oder Plasma, der einen innen hohlen zylindrischen Behälterkörper (2) umfasst, der an seinem Einlassende (2a) und seinem Auslassende (2b) mit entsprechenden Zugangsöffnungen (3, 4) versehen ist, die in Abschlusskappen (5, 6) ausgebildet sind, und in seinem Inneren ein Filtermedium (7) enthält, das aus mikroporösen Fasern (8) mit offenen Enden aufgebaut ist, die in mehreren konzentrisch als Rolle gewickelten Schichten angeordnet sind und die Diethylaminoethylgruppen tragen, **dadurch gekennzeichnet, dass** im Filter nur ein einziges Filtermedium (7) enthalten ist, das aus mikroporösen Fasern (8) aufgebaut ist, und dass ausschließlich am Auslassende (2b) des Körpers (2) ein Packungselement (9) zum stabilen Bündeln der Enden der Fasern (8) vorgesehen ist, wobei die offenen Enden sämtlicher mikroporöser Fasern (8) am Auslassende (2b) des Körpers (2) im Packungselement (9) eingebettet sind und durch dieses hindurchtreten.

2. Adsorptionsfilter nach Anspruch 1, **dadurch gekennzeichnet, dass** die Zugangsöffnungen (3, 4) für das Einströmen (3) beziehungsweise Ausströmen (4) von Blut und/oder Plasma in den Kappen (5, 6) so angeordnet sind, dass sie in Bezug auf den Körper (2) zentriert und koaxial sind, wobei eine Schnellablassöffnung (10) an dem Körper (2) in der Nähe des Auslassendes (2b) angeordnet ist.

3. Adsorptionsfilter nach Anspruch 1, **dadurch gekennzeichnet, dass** eine zylindrische Längsstrebe (11) im Hohlraum (2c), der von dem Körper (2) gebildet wird, koaxial zentriert angeordnet ist, die zum Bewickeln und zum Stützen des Filtermediums (7) aus mikroporösen Fasern bestimmt ist.

4. Adsorptionsfilter nach Anspruch 1 und 3, **dadurch gekennzeichnet, dass** das aus mikroporösen Fasern bestehende Filtermedium (7) aus einer gewebten Membranbahn (12) gebildet ist, deren Schussfäden aus den mikroporösen Membranen (12) bestehen, die parallel verlaufen und die zickzackartig gefaltet, für einen festen Sitz in dem axialen Hohlraum (2c) des Behälterkörpers (2) in Rollenform gebündelt und auf der Strebe (11) aufgesteckt sind.

5. Adsorptionsfilter nach Anspruch 4, **dadurch gekennzeichnet, dass** die gewebte Membranbahn (12) mit den Schussfäden aus parallelen mikroporösen Fasern rechteckig ist und mit mindestens einer zweiten rechteckigen gewebten Bahn (8a) gepaart ist, die im Wesentlichen identisch ist und aus einem Material mit Schussfäden aus parallelen nicht porösen Fasern hergestellt ist, wobei beim Bündeln der gewebten Bahn (12) in Rollenform eine abwechselnde konzentrischer Schichtung mikroporöser Fasern (8) und nicht poröser Fasern (8a) bewirkt wird.

6. Adsorptionsfilter nach Anspruch 4, **dadurch gekennzeichnet, dass** die gewebte Membranbahn (12) mit Schussfäden aus parallelen mikroporösen Fasern rechteckig ist und mit mindestens einer zweiten identischen gewebten Bahn (8a) gepaart ist, wobei durch das Bündeln der gewebten Bahnen (12) in Rollenform eine abwechselnde konzentrische Schichtung mikroporöser Fasern (8) bewirkt wird.

7. Adsorptionsfilter nach den vorhergehenden Ansprüchen, **dadurch gekennzeichnet, dass** zumindest die parallelen mikroporösen Fasern (8), die die Schussfäden der gewebten Membranbahn (12) darstellen, unter einem Winkel zur Längsachse des Behälterkörpers (2) angeordnet sind, wobei durch das Bündeln in Rollenform ein Filtermedium (7) mit sich gitterartig kreuzenden geschichteten mikroporösen Fasern (8) erzeugt wird.

8. Adsorptionsfilter nach den vorhergehenden Ansprüchen, **dadurch gekennzeichnet, dass** die mikroporösen Fasern (8) aus einem Kunststoffmaterial ausgewählt aus Polyamid, Polysulfon, Polyethersulfon, Polyethylen, Polypropylen und Polyester bestehen.

9. Adsorptionsfilter nach Anspruch 6, **dadurch gekennzeichnet, dass** die gewebte Bahn (12) mit Schussfäden aus mikroporösen Fasern (8) entlang der Längsachse (B) senkrecht zu den Fasern (8) umgeklappt ist.

10. Adsorptionsfilter nach den vorhergehenden Ansprüchen, **dadurch gekennzeichnet, dass** die parallelen nicht porösen Fasern (8a) unter einem Winkel zur Achse (A) des Behälterkörpers (2) angeordnet sind, sodass sie den mikroporösen Fasern (8) spiegelsymmetrisch gegenüberliegen.

11. Adsorptionsfilter nach den vorhergehenden Ansprüchen, **dadurch gekennzeichnet, dass** die nicht porösen Fasern (8a) aus einem Polymermaterial ausgewählt aus Polyethylen, Polypropylen, Polyester oder Nylon bestehen.

12. Adsorptionsfilter nach den vorhergehenden Ansprüchen, **dadurch gekennzeichnet, dass** die mikroporösen Fasern (8) und die nicht porösen Fasern (8a) unter einem Winkel asymmetrisch entgegengesetzt angeordnet sind.

13. Adsorptionsfilter nach den vorhergehenden Ansprüchen, **dadurch gekennzeichnet, dass** die Durchmesser der mikroporösen Fasern (8) und der nicht porösen Fasern (8a) miteinander identisch sind.

14. Adsorptionsfilter nach den vorhergehenden Ansprüchen, **dadurch gekennzeichnet, dass** sich die Durchmesser der mikroporösen Fasern (8) und der nicht porösen Fasern (8a) voneinander unterscheiden.

15. Adsorptionsfilter nach Anspruch 6 und 7, **dadurch gekennzeichnet, dass** die Schrägstellung der mikroporösen Fasern (8) und der nicht porösen Fasern (8a) erfolgt, nachdem vor dem Bündeln in die Rollenform auf mindestens zwei diagonale Enden der gewebten Bahnen (12) eine Zugkraft aufgebracht wurde.

16. Adsorptionsfilter nach den vorhergehenden Ansprüchen, **dadurch gekennzeichnet, dass** das Filtermedium (7), das in Rollenform gebündelt ist, eine aus mikroporösen Fasern (8) gebildete Umfangsfläche aufweist.

17. Adsorptionsfilter nach Anspruch 1, **dadurch gekennzeichnet, dass** das Element zum stabilen Bündeln durch ein statisches Element zur Flusskontrolle (13) gebildet ist, das aus einem Elastomer hergestellt ist und in das die offenen Enden der mikroporösen Fasern (8) so eingebettet sind, dass sie durch es hindurchtreten, wobei das Element zur Flusskontrolle (13) in das Auslassende (2b) des axialen Hohlraums (2c) des zylindrischen Behälterkörpers (2) eingepasst ist.

18. Adsorptionsfilter nach Anspruch 1 und 14, **dadurch gekennzeichnet, dass** ein zweites statisches Element zur Flusskontrolle (13) aus einem Elastomer auch im Einlassende (2a) des axialen Hohlraums (2c) des zylindrischen Behälterkörpers (2) angeordnet ist.

19. Adsorptionsfilter nach Anspruch 1, 14 und 15, **dadurch gekennzeichnet, dass** entsprechende Auffangkammern (14, 15) zwischen den Kappen (5, 6) und dem statischen Element zur Flusskontrolle (13) und den Umkehrbögen der mikroporösen Fasern (8) gebildet sind.

## Revendications

1. Filtre pour l'adsorption d'héparine et/ou d'endotoxines dans le sang et/ou le plasma, comprenant un corps de confinement cylindrique et intérieurement creux (2) qui est pourvu, à ses extrémités d'entrée (2a) et de sortie (2b), d'orifices d'accès correspondants (3, 4) formés dans des capsules d'extrémité (5, 6) et recevant à l'intérieur un milieu filtrant (7) composé de fibres microporeuses (8) ayant des extrémités ouvertes et étant agencées en plusieurs couches qui sont enroulées de façon concentrique en rouleau et sur lesquelles sont déposés des groupes de diéthylaminoéthyl, **caractérisé en ce que** le filtre contient un seul milieu filtrant (7) composé uniquement de fibres microporeuses (8) et **en ce qu'**un élément de remplissage (9) pour le calage stable des extrémités des fibres (8) est prévu à l'extrémité de sortie (2b) seulement dudit corps (2), dans lequel les extrémités ouvertes de toutes les fibres microporeuses (8) sont noyées à l'extrémité de sortie (2b) dudit corps (2) dans l'élément de remplissage (9) de manière à passer à travers celui-ci.

2. Filtre adsorbant selon la revendication 1, **caractérisé en ce que** lesdits orifices d'accès (3, 4), respectivement pour le flux entrant (3) et pour le flux sortant (4) de sang et/ou de plasma, sont agencés dans lesdites capsules (5, 6) de manière à être centrés et coaxiaux par rapport audit corps (2), un orifice d'évacuation rapide (10) étant placé dans ledit corps (2) à proximité de l'extrémité de sortie (2b).

3. Filtre adsorbant selon la revendication 1, **caractérisé en ce qu'**une nervure longitudinale cylindrique (11) est centrée de façon coaxiale à l'intérieur de la cavité (2c) formée par ledit corps (2) et est destinée à l'enveloppement et au support dudit milieu filtrant à fibres microporeuses (7).

4. Filtre adsorbant selon les revendications 1 et 3, **caractérisé en ce que** ledit milieu filtrant à fibres microporeuses (7) est constitué par une feuille de membrane tissée (12) ayant une trame composée desdites membranes microporeuses (12), qui sont parallèles et pliées en zigzag, emballée sous la forme d'un rouleau pour une mise en place serrée dans ladite cavité axiale (2c) dudit corps de confinement (2) et montée sur ladite nervure (2).

5. Filtre adsorbant selon la revendication 4, **caractérisé en ce que** ladite feuille tissée de membrane (12) avec une trame à fibres microporeuses parallèles est rectangulaire et est couplée à au moins une deuxième feuille tissée rectangulaire (8a) qui est substantiellement identique et faite d'un matériau ayant une trame de fibres non poreuses parallèles, l'emballage de ladite feuille tissée (12) sous la forme d'un rouleau provoquant une mise en couches concentriques alternées de fibres microporeuses (8) et de fibres non poreuses (8a).

6. Filtre adsorbant selon la revendication 4, **caractérisé en ce que** ladite feuille tissée de membrane (12) avec une trame à fibres microporeuses parallèles est rectangulaire et est couplée à au moins une deuxième feuille tissée identique (8a), l'emballage desdites feuilles tissées (12) sous la forme d'un rouleau provoquant une mise en couches concentriques alternées de fibres microporeuses (8).

7. Filtre adsorbant selon les revendications précédentes, **caractérisé en ce que** au moins lesdites fibres microporeuses parallèles (8) qui constituent la trame de ladite feuille tissée de membrane (12) sont agencées en formant un angle par rapport à l'axe longitudinal dudit corps de confinement (2), ledit emballage sous la forme d'un rouleau générant ledit milieu filtrant (7) avec des fibres microporeuses stratifiées (8) croisées à la façon d'une grille.

8. Filtre adsorbant selon les revendications précédentes, **caractérisé en ce que** lesdites fibres microporeuses (8) sont faites d'une matière plastique choisie parmi le polyamide, le polysulfone, le polyéthersulfone, le polyéthylène, le polypropylène et le polyester.

9. Filtre adsorbant selon la revendication 6, **caractérisé en ce que** ladite feuille tissée (12) à trame en fibres microporeuses (8) est repliée le long de l'axe longitudinal (B) perpendiculaire aux fibres (8).

10. Filtre adsorbant selon les revendications précédentes, **caractérisé en ce que** lesdites fibres non poreuses parallèles (8a) sont agencées en formant un angle par rapport à l'axe (A) dudit corps de confinement (2) de manière à être opposées, de façon symétrique, par rapport auxdites fibres microporeuses (8).

11. Filtre adsorbant selon les revendications précédentes, **caractérisé en ce que** lesdites fibres non poreuses (8a) sont faites d'un matériau polymère choisi parmi le polyéthylène, le polypropylène, le polyester et le nylon.

12. Filtre adsorbant selon les revendications précédentes, **caractérisé en ce que** lesdites fibres microporeuses (8) et lesdites fibres non poreuses (8a) sont agencées en formant un angle de façon opposée et asymétrique.

13. Filtre adsorbant selon les revendications précédentes, **caractérisé en ce que** les diamètres desdites fibres microporeuses (8) et desdites fibres non poreuses (8a) sont mutuellement identiques.

14. Filtre adsorbant selon les revendications précédentes, **caractérisé en ce que** les diamètres desdites fibres microporeuses (8) et desdites fibres non poreuses (8a) sont mutuellement différents.

15. Filtre adsorbant selon les revendications 6 et 7, **caractérisé en ce que** l'inclinaison desdites fibres microporeuses (8) et desdites fibres non poreuses (8a) est réalisée après une traction appliquée à au moins deux extrémités diagonales desdites feuilles tissées (12) avant d'être conditionné sous la forme d'un rouleau.

16. Filtre adsorbant selon les revendications précédentes, **caractérisé en ce que** ledit milieu filtrant (7) emballé sous la forme d'un rouleau a une surface périmétrique constituée par de la fibre microporeuse (8).

17. Filtre adsorbant selon la revendication 1, **caractérisé en ce que** ledit élément d'emballage stable est constitué par un élément de commande de débit statique (13) fait d'un élastomère dans lequel lesdites extrémités ouvertes des fibres microporeuses (8) sont noyées afin de passer à travers celui-ci, ledit élément de commande de débit (13) étant inséré de façon serrée dans l'extrémité de sortie (2b) de la cavité axiale (2c) dudit corps de confinement cylindrique (2).

18. Filtre adsorbant selon les revendications 1 et 14, **caractérisé en ce qu'**un deuxième élément de commande de débit statique (13) fait d'un élastomère est aussi inséré dans l'extrémité d'entrée (2a) de la cavité axiale (2c) dudit corps de confinement cylindrique (2).

19. Filtre adsorbant selon les revendications 1, 14 et 15, **caractérisé en ce que** des chambres de collecte correspondantes (14, 15) sont formées entre lesdites capsules (5, 6) et ledit élément de commande de débit statique (13) et des courbures de retour desdites fibres microporeuses (8).
